# EUROPEAN PATENT SPECIFICATION

(11) **EP 3 558 148 B1**
(45) Date of publication and mention of the grant of the patent: **16.11.2022**
(21) Application number: 17887432.7
(22) Date of filing: 22.12.2017
(51) Int. Cl.: A61B 18/04, A61B 17/16, A61B 17/17

(54) **SURGICAL NAVIGATION USING A GUIDE FOR INSTRUMENTATION POSITIONING**
CHIRURGISCHE NAVIGATION MIT EINER FÜHRUNG ZUR INSTRUMENTENPOSITIONIERUNG
NAVIGATION CHIRURGICALE À L'AIDE D'UN GUIDE POUR POSITIONNEMENT D'INSTRUMENTATION

(30) Priority: 26.12.2016 US 201662439094 P
(43) Date of publication of application: 30.10.2019
(73) Proprietor: Marino, James F., La Jolla, CA 92037 (US); Elbanna, Jamil, San Diego, CA 92128 (US)
(72) Inventor: Marino, James F., La Jolla, CA 92037 (US); Elbanna, Jamil, San Diego, CA 92128 (US)
(74) Representative: Slingsby Partners LLP
(86) International application number: PCT/US2017/068303
(87) International publication number: WO 2018/125834

(56) References cited:
- EP-A1- 2 505 165
- EP-A1- 2 712 585
- WO-A2-2004/089192
- US-A- 6 167 145
- US-A1- 2008 051 910
- US-A1- 2010 286 713
- US-A1- 2010 298 834
- US-A1- 2014 107 471
- US-A1- 2014 276 870
- US-B2- 8 712 503

## Description

### BACKGROUND

Before the present subject matter is further described, it is to be understood that this subject matter described herein is not limited to particular embodiments described, as such may of course vary. It is also to be understood that the terminology used herein is for the purpose of describing particular embodiments only, and is not intended to be limiting. Unless defined otherwise, all technical terms used herein have the same meaning as commonly understood by one skilled in the art to which this subject matter belongs.

Surgical procedures requiring high precision of instrumentation and/or implant placement are increasingly performed with the aid of various surgical navigation devices. Some of these devices utilize computer generated three-dimensional (3-D) modeling of relevant anatomy combined with intraoperative radiologic or electromechanical (infrared or radiofrequency) registration (i.e., fiduciaries), to locate the relevant anatomy in space. While these systems may be versatile, they often require expensive and potentially harmful (e.g., ionizing irradiation from CT scans) imaging studies, as well as placing significant intraoperative demands for registration.

A goal for surgical navigation is to spatially position instrumentation and/or implants accurately with respect to certain anatomy of the patient (e.g., acetabular component of a hip replacement procedure within the acetabular socket). At times, the relevant anatomy can be oriented in space based upon surface landmarks that might be accessible via direct or indirect inspection and/or palpation of the patient (e.g., the anterior superior iliac spines and the symphysis pubis, defining the anterior pelvic plane). At other times, an instrument can be placed on or within the skeletal anatomy (e.g., intramedullary rod with condylar referencing) to provide skeletal spatial positioning. Thus, tools to assist in the accurate placement of instrumentation and/or implants are desired.

US8712503 discloses a device for registering in a medical navigation system a position and/or orientation of a patient's pelvis which includes a pelvis registration frame. A plurality of positioning elements are arranged on the pelvic registration frame so as to define a plane, wherein the plurality of positioning elements are moveable with respect to each other to enable placement of the positioning elements at defined pelvic points of the patient's pelvis. At least one position transmitter is arranged in an unambiguously defined position and/or orientation relative to the frame.

### SUMMARY

The present invention is directed to a device according to claim 1.

Aspects of the current subject matter relate to surgical navigation tools.

Aspects of the current subject matter allow for assessment of soft tissue depth beneath stanchions of an anterior pelvic frame, allowing for adjustment of the frame to optimize pin/screw placement in the hemi-pelvis in a manner that accurately correlates with the boney anatomy that defines the anterior pelvic plane.

Additional aspects relate to a defined specific relationship between two skeletally fixed pins/screws to the hemi-pelvis and the anterior pelvic frame. Tools utilizing two screws with this specific spatial relationship enable a method of referencing the anterior pelvic plane to be performed, as well as determining limb length and trochanteric offset. Additional aspects provide controlled acetabular depth and orientation of reaming with the aid of the pins/screws fixed in a spatially known relationship to the anterior pelvic frame.

### DESCRIPTION OF DRAWINGS

The accompanying drawings, which are incorporated in and constitute a part of the specification, show certain aspects of the subject matter disclosed herein and, together with the description, help explain some of the principles associated with the disclosed implementations. In the drawings:
FIGs. 1-8 illustrate various features of a frame, a reference sensor, a positioning sensor, and a mechanical drill guide consistent with implementations of the current subject matter; and
FIGs. 9-12 illustrate features of pin/screw assemblies that can be used with various implementations of the current subject matter.

When practical, similar reference numbers denote similar structures, features, or elements.

### DETAILED DESCRIPTION

While there are various existing means of utilizing surgical navigation, generally with computer assistance (e.g., computer assisted surgery (CAS) or robotic navigation), these have had sufficient detractions in cost, effectiveness, and/or convenience to limit their utilization and utility. The disclosed devices overcome at least some of these detractions, by utilizing relatively low cost means (wireless communicating multiaxial inclinometers and compass MEM sensors and/or mechanical surgical guides and surgical guide rods engaged) of spatially referencing relevant skeletal anatomy relative to instrumentation used to fashion bone or position implants, for example.

The disclosed devices relate to fixing (i.e., positioning) a multiaxial reference sensor (e.g., inclinometer(s) and compass sensor) or a mechanical guide to the skeletal anatomy in a known orientation, and then utilizing this reference sensor or mechanical guide to position instrumentation and/or implants with a second multiaxial positioning sensor or via a guide rod that provides spatial positioning information relative to the reference sensor or skeletally fixed references.

For example, in performing hip replacement surgery, it is highly desirable to orient the acetabular component in a specific manner that optimizes subsequent implant stability, limb strength, limb length, and implant surface wear. The most common method for ascertaining this is based upon surgeon experience and the use of skeletal (e.g., acetabular rim) and soft tissue (e.g., transverse acetabular ligament) landmarks. While this method can be very accurate in general, there remains a small but significant percentage of patients who will have implants positioned sub-optimally, leading to higher rates of dislocation, implant impingement, relative weakness of the limb musculature, increased implant surface wear rates, and symptomatic limb length inequalities.

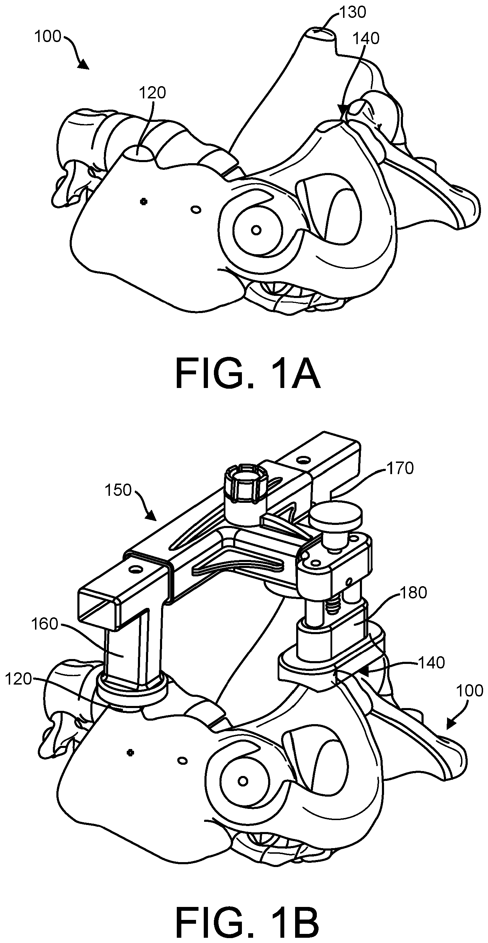

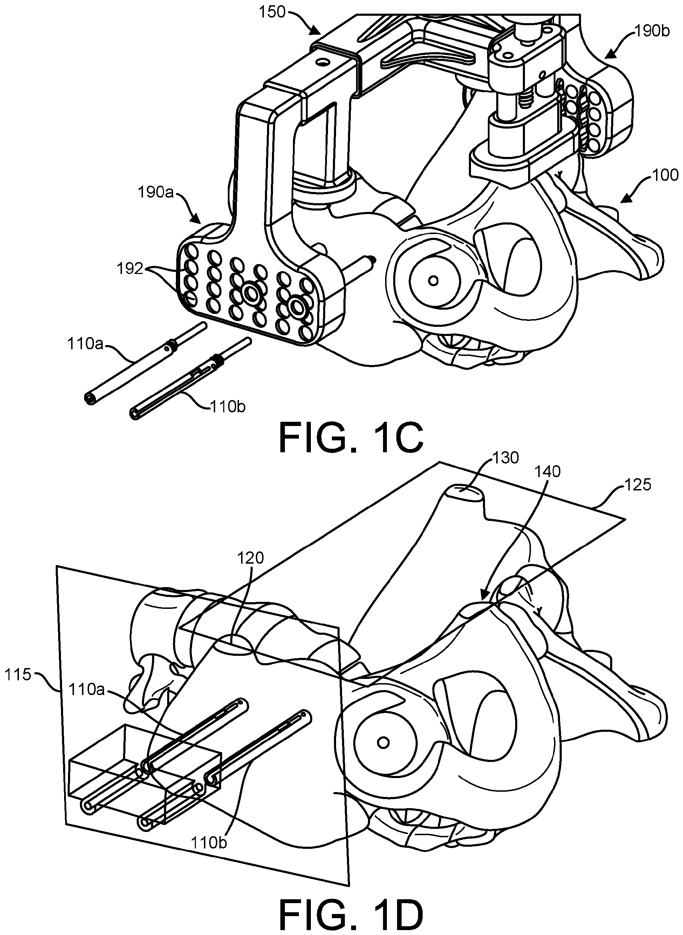

FIG. 1A illustrates a pelvis 100 of a supine patient indicating the anterior superior iliac spine (ASIS) prominences 120,130 and the symphysis pubis (SP) 140. With reference to FIG. 1B, in accordance with an embodiment, a three-legged frame 150 is positioned over the pelvis 100 of the supine patient, with each of two legs 160,170 resting on the cutaneous surfaces immediately superficial to the anterior superior iliac spine (ASIS) prominences 120,130, while a third leg 180 is supported by the skin and subcutaneous tissue immediately superficial to the symphysis pubis (SP) 140. The frame 150 is configured to maintain the distance from each ASIS to the SP, equidistant.

As shown in FIG. 1C, one or more adjustable surgical guides 190a, 190b are associated with the frame 150. The guides 190a, 190b are configured to position pins or screws (e.g., two pins 110a, 110b, as shown in FIG. 1C) in the lateral pelvis superior to the acetabular cavity. The guides 190a, 190b may allow for additional pins or screws to be positioned. The guides 190a, 190b comprise one or more guide holes or channels 192 for such positioning and placement.

The two specialized pins/screws 110a,110b are placed into the lateral aspect of the pelvis above the acetabular fossa (as shown in FIG. 1C). These pins/screws 110a,110b are inserted along axes that are each parallel with a line defined by both anterior superior iliac spine (ASIS) prominences 120,130. As shown in FIG. ID, a plane 115 that contains bone pins/screws' axes is parallel with a plane defined by both ASISs 120,130 and the SP 140 or is referenced to the anterior pelvic plane 125 (defined by the symphysis pubis (pubic tubercles) 140 and both ASISs 120,130).

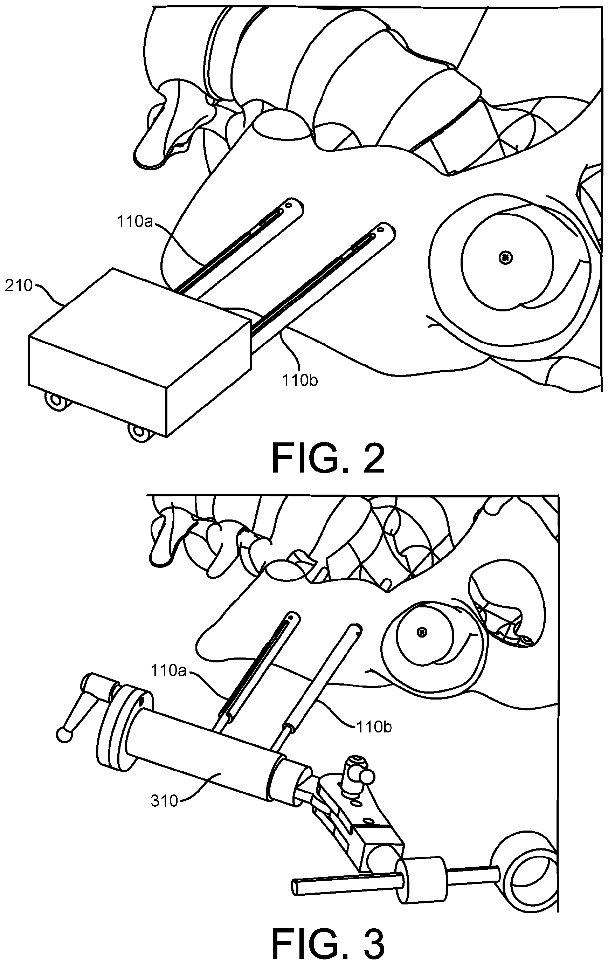

Now with reference to FIG. 2, a first multiaxial sensor 210 (i.e., a reference sensor) is attached to the two pins/screws 110a,110b, such that when the patient is placed on his or her side (positioned for surgery) the reference sensor 210 is located above the patient's pelvis 100. If the patient is positioned supine for the hip replacement procedure, the reference sensor 210 is positioned lateral to the respective operative hemi-pelvis. Alternatively, as shown in FIG. 3, a mechanical drill guide 310 or reamer/implant insertor guide rod may be temporarily associated with both pins/screws 110a,110b, to provide orientation for acetabular drilling, reaming, and implant insertion. As shown in FIG. 3, the drill guide 310 has an attached radially positioned ring configuration (attached via a connection component or structure) to visually position a drill within the acetabular socket of the patient.

As described above, the pins/screws 110a,110b are placed into the pelvis 100 such that a plane containing both of their axes is parallel (or angularly referenced) to the plane defined by the two ASISs 120,130 and the symphysis pubis 140 or anterior prominences of the pubic tubercles (i.e., the anterior pelvic plane). Each axis of the pin/screw 110a,110b is parallel to a line defined by both ASISs 120,130. Thus, the reference sensor 210, or mechanical drill guide and guide rod positioner 310, is positioned and secured to the patient's pelvis in a manner that relative orientation to the reference sensor 210, or to the mechanical drill guide and guide rod positioner 310, provides relative orientation to the pelvis (via their collective spatial reference to the anterior pelvic plane).

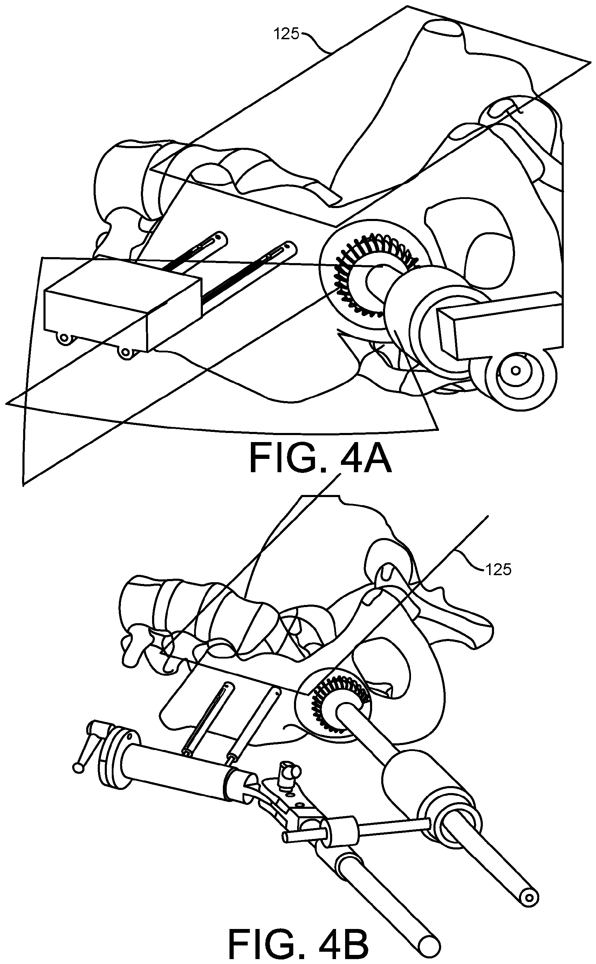

With reference to FIGs. 4A and 4B, reference abduction and anteversion values can be determined and/or recorded relative to a reference multiaxial sensor 210 or a fixed or adjustable mechanical drill/reamer/insertor guide 310 associated with the two screws/pins 110a,110b having axes oriented parallel to the anterior pelvic plane as well as parallel to a line defined by both ASISs 120,130. In a lateral decubitus position, an MEM sensor (i.e., inclinometer) records coronal plane pelvic tilt reference values determined by the angle subtended by the inclinometer relative to the gravitational vector within a plane parallel angularly referenced to the anterior pelvic plane (also parallel to a plane defined by the axes of the two pins/screws 110a,110b).

In this same lateral position, the reference anteversion values are recorded by an inclinometer sensor measuring within a plane that is orthogonally intersected by the sagittal plane or a plane. A third sensor (a heading or magnetic field sensor) is orthogonally oriented relative to the previous two MEM sensors, with possible utilization to adjust for axial plane tile variance relative to the sagittal plane (determined, for example, through the use of preoperative or intraoperative X-rays). The sensor is optional as there are no critical values to be determined in this plane. If the patient is positioned in the supine position, one of the inclinometer sensors will exchange functionality with the magnetic field or heading sensor.

With the multiaxial reference sensor 210 secured to the pelvis via two pins or screws 110a,110b and used to determine the orientation of the pelvis in space, use of a second multiaxial measurement sensor associated with the instrumentation used to fashion the pelvis (e.g., reamer shaft) or insert the acetabular implant (e.g., cup positioner shaft), enables the determination of relative spatial orientation (that is relative to the reference sensor 210) that provides for relatively precise and objective acetabular floor (i.e. foveal or cotyledon notch) drilling, acetabular reamer orientation, and implant position orientation, relative to the pelvic anatomy. Similarly, a mechanical drill guide/guide rod positioner 310 (fixed or adjustable) can be temporarily associated with the two pins/screws110a,110b (fixed to the hemi pelvis), to provide for precisely directed drilling of the acetabular floor (to assess and control for acetabular reaming depth, reamer position-relative to the native acetabular socket, and reamer/implant orientation-relative to the anterior pelvic plane).

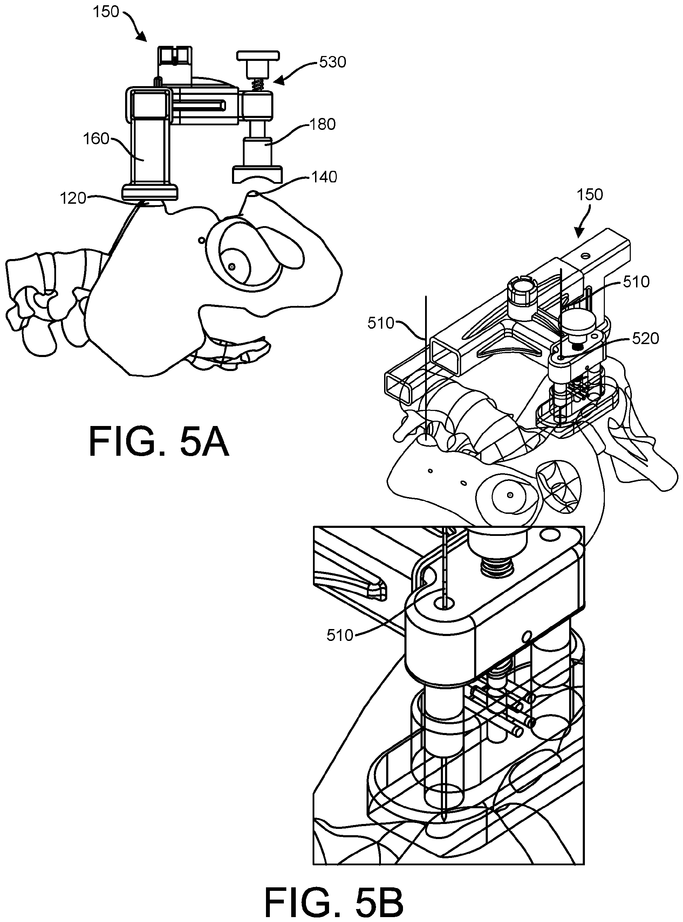

With reference to FIGs. 5A and 5B, with the patient in a supine position, and the operative (e.g., arthritic hip scheduled for replacement) positioned at the edge of the operative table (to allow the gluteal mass to fall posteriorly), the patient's anterior pelvic plane is determined by palpation of the subcutaneous boney landmarks (i.e., both ASISs 120,130 and the SP 140). An adherent localizing skin marker (e.g., EKG electrode or something similar) can be placed on the relaxed skin immediately over the boney prominence. These skin markers can be used to localize as well as stabilize the three supportive stanchions or legs 160,170,180 of the pelvic frame 150. Alternatively, the stanchions 160,170,180 can rest on the surface anatomy with conforming surface features to reduce the risk of frame migration. Measurement of the soft tissue depth (i.e., the distance from the skin surface immediately below the stanchions or legs 160,170,180 to the underlying bone) superficial to one or each ASIS 120,130 and the pubic tubercle(s), is accomplished with the use of one or more pin depth probes 510 (e.g. measured from the top of each stanchion 160,170,180 to the superficial underlying bone) or via a non-invasive ultrasound diagnostic measurement tool. This can be accomplished via a relatively small notch or aperture/channel 520 in the stanchions. The disparity in soft tissue depth associated with the stanchion supports can be accommodated via an adjustment in height of one or several of the stanchions 160,170,180 (it is anticipated that the soft tissue depth associated with both ASISs will be similar and the disparity between the soft tissue depth associated with the pubic tubercle stanchion and the ASIS stanchions will be of greater clinical concern). In an embodiment, a single height adjustment component 530 is provided to adjust the height of the symphysis pubis stanchion 180, to accommodate for this variance in soft tissue depth that can lead to inaccuracies of representation of the anterior pelvic plane.

The pelvic frame 150 is positioned over the respective boney landmarks of the ASISs 120,130 and the symphysis pubis (pubic tubercles) 140. This can be accomplished in a supine position or in the lateral decubitus position. Compression of the frame 150 against the patient is accomplished via, for example, an attachment to the operative table rail to either compress the pelvis against the operative table padding (i.e. supine position) or against a sacral support pad (i.e. lateral decubitus positioning). A spring loaded compression mechanism can be integral to the pelvic frame positioner. A generous area of skin immediately adjacent and superior to the operative side greater trochanter is prepped with a rapid acting antiseptic skin preparation and field towels used to isolate the area from adjacent regions. A sterile-gloved or gloved and gowned surgeon then secures the pin/screw guide 190a,190b to a track on the operative side and then slides the pin guide 190a,190b along the track until the pin guide 190a,190b is adjacent to the proximal lateral thigh and pelvis. Adjustments for the position of the frame 150 and the attached drill/pin/screw guide 190a,190b can be secured with friction locks or the like. Small stab incisions are made into the skin a prescribed distance above the greater trochanter (e.g., two finger breadths), and two pins or screws 110a,110b are advanced into the pelvis along the axes defined by the sterile guide 190a,190b, with or without the aid of a sharp drill or trocar tip to create cortical holes aligned with the pin guides channels 192.

The pins or screws 110a,110b are then advanced into the pelvis such that they are secured to both the lateral and the medial wall of the ilium, with axes parallel to both the anterior pelvic plane and a line segment defined by both ASISs 120,130. The pins or screws 110a, 110b may reside essentially flush with or sub-flush with the soft tissue mass of the lateral pelvis. A skin sterile barrier may then be placed over the pin/screw tract wounds and the patient may then be positioned and secured as would normally occur for a hip replacement procedure (i.e., generally in the lateral decubitus position with the operative side up), unless the pelvic frame itself is being used with appropriate padding attachments for lateral decubitus positioning. Standard preparation and draping would ensue (adherent skin sterile barrier removed before or after this effort).

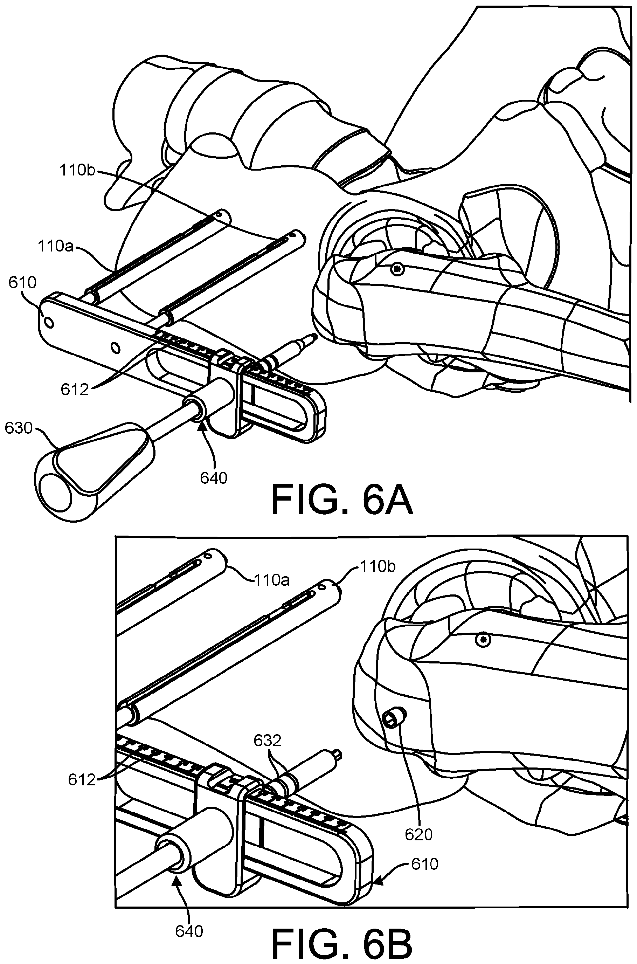

With reference to FIGs. 6A and 6B, after exposing the hip but prior to hip dislocation, a guide 610 is associated with both pins 110a,110b, to define an axis that extends over the greater trochanter. With the operative limb positioned generally along the axis of the guide 610 and maintained in neutral abduction/adduction, a screw 620 with deeply recessed hex form or similar channel for association with a screw driver 630 is advanced through a sliding drill/screw guide channel 640 associated with and orthogonal to the guide axis and generally parallel to the two pelvic pins/screws 110a, 110b. The axial distance between the trochanteric reference screw 620 and the lower pelvic pin/screw 110b (or a surrogate distance) is noted from graduations 612 on the guide 610. This value is used for comparison to subsequent measurements to determine limb length changes. Additionally, the depth from an axis of the guide 610 to the screw head 620 is measured using graduations 632 on the screw driver shaft. This value can be used for assessing changes in the "hip offset", following implant trialing or implantation.

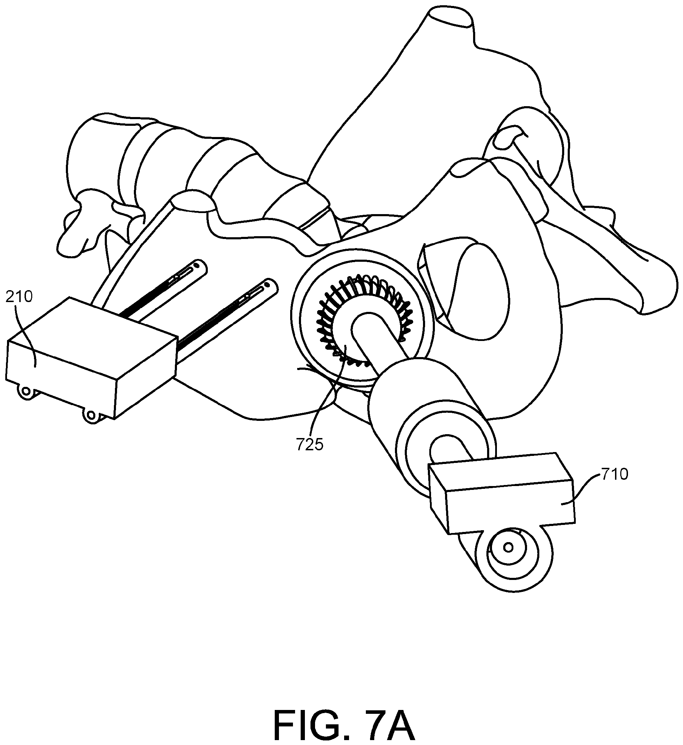

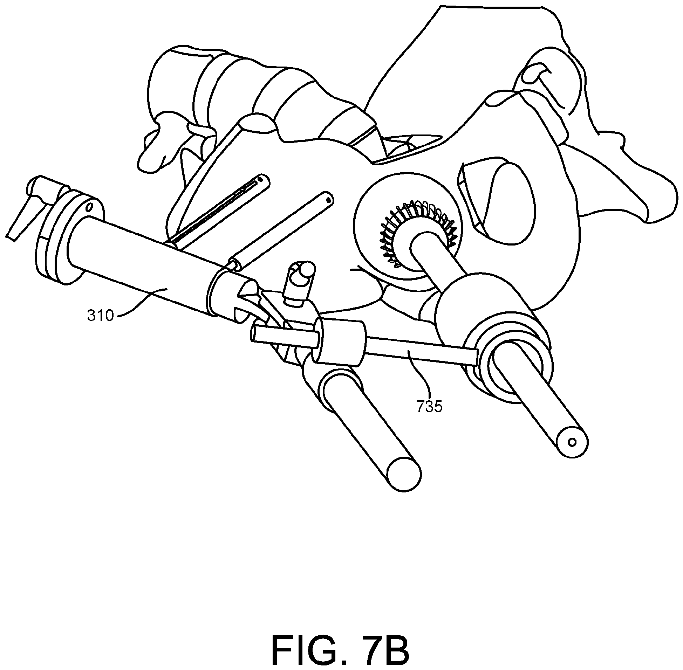

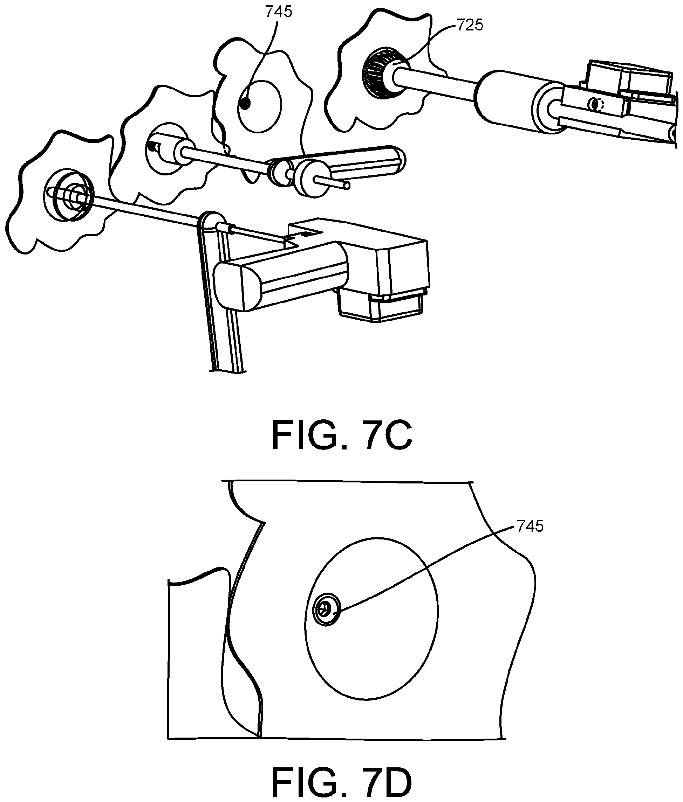

After this is completed, the guide 610 is removed and the hip is dislocated and fully prepared for acetabular reaming. With reference to FIGs. 7A-7D, the sterile wireless multiaxial reference sensor 210 (FIG. 7A) or mechanical drill guide 310 (FIG. 7B) is then secured to the two pins/screws 110a,110b in the pelvis 100 (e.g., with the aid of a thread-form that engages either an internal or external feature of the pins/screws). As shown in FIG. 7A, a second measurement sterile wireless multiaxial sensor 710 can be fixed to the acetabular floor drill or drill guide, final reaming shafts or reamer, and acetabular cup positioner, should drilling and reaming orientation relative to the pelvis be desired and the mechanical axis guide is not employed. A acetabular cup 725 is positioned with the aid of a measurement multiaxial sensor 220 to achieve optimal acetabular abduction and anteversion. Alternatively, as shown in FIG. 7B, a guide rod 735 can be oriented via the mechanical guide 310 (associated with the pins/screws 110a,110b in the hemi pelvis) and fixed to the operative table via an adjustable positioner and to the pelvis via a foveal or cotyledon notch/pelvic floor screw 745. The pelvic floor screw 745 is inserted into a pre-drilled hole in the floor of the acetabulum that is drilled in alignment with the intended reaming/implant axis (i.e., intended final inclination and anteversion). Based upon preoperative and intraoperative planning the "flat head" of acetabular floor screw 745 is positioned at a prescribed depth to control reaming depth. A central recess in the screw head accommodates the reamer guide rod/pin and implant insertion guide rod to constrain both reaming and implant insertion along a predetermined optimized axis. In accordance with an embodiment, the acetabular floor may be "spot faced" to a specific depth, such that the acetabular floor screw head, when inserted to the depth of the spot faced acetabular surface, prevents excessive acetabular floor reaming by serving as a mechanical stop to cannulated reamers. Markings on the reamer and insertion guide rod (visible above the cannulated reamer and cannulated implant insertor) provide additional visual confirmation of depth of acetabular reaming and implant insertion.

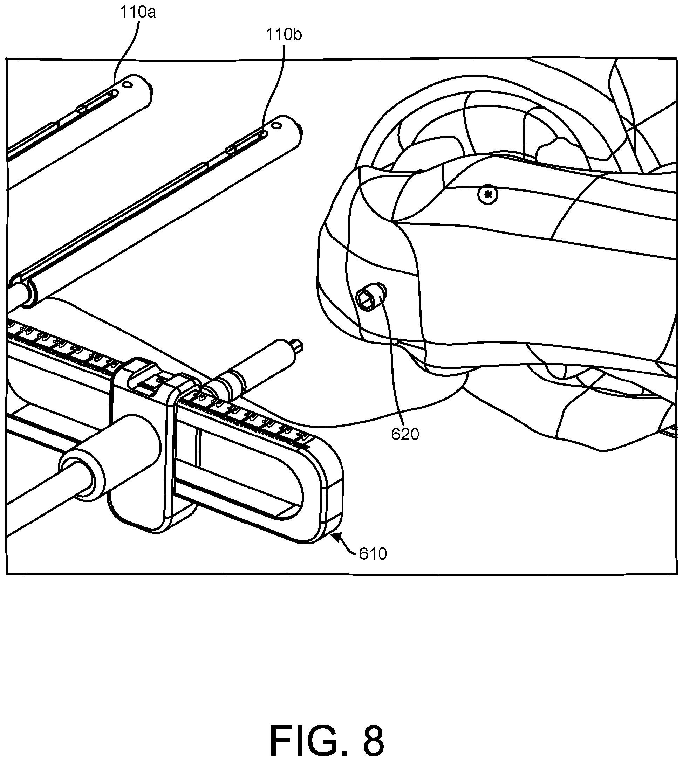

Trialing of the femoral component located within the acetabular socket with the use of the axial guide 610, and screw driver inserter associated with the previously inserted trochanteric screw 620, as shown in FIG. 8, allows for comparison and adjustment of anticipated limb length and "hip offset" (as long as the limb is returned to neutral abduction/adduction).

A method of securing the wireless reference sensor (as well as the leg length and acetabular offset measuring instruments) to the pelvis is now described. Use of relatively conventional trocar tipped Steinman pins or bone screws of various types can be used, but a more predictable means of rapidly and securely fixating to both the outer and inner tables of the pelvis, percutaneously is also disclosed.

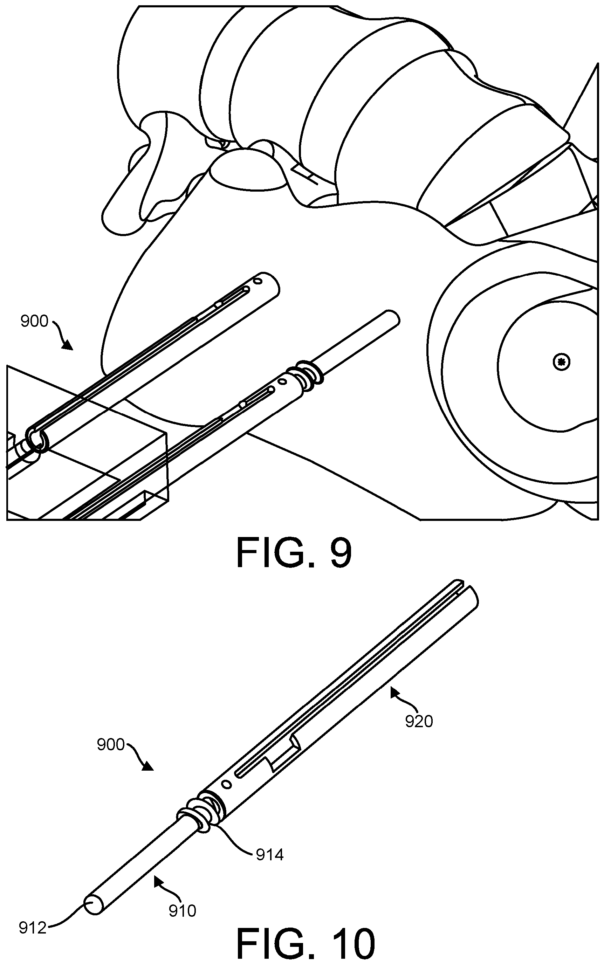

The pin-screw assembly 900 described below is an alternative to conventional pins or screw forms. The segmented pelvic pin and screw combination 900 is shown in FIGs. 9-11 in various disassembled and assembled or engaged forms. The assembly 900 includes a distal segment 910 having a piercing tip 912 as well as having a trailing or proximal external male thread form 914 and either an internal (shown in this illustrated example) or external geometric (e.g. hex, square, and Torx) form for forward advancement (driving) and rotating the combined assembly.

A proximal segment 920 of the pin-screw assembly 900 includes a cannulated element 922 having an internal (female) thread form (to compliment the male thread form 914 of the distal pin component) and an external bone thread having an identical thread pitch. In addition, the proximal component 920 has a torsional drive feature, such as the external hex drive feature shown.

A cannulated torsion drive can be used to advance the proximal threaded segment 920 on to the distal pin segment 910, while the pin handle is utilized to provide counter rotational resistance to the pin.

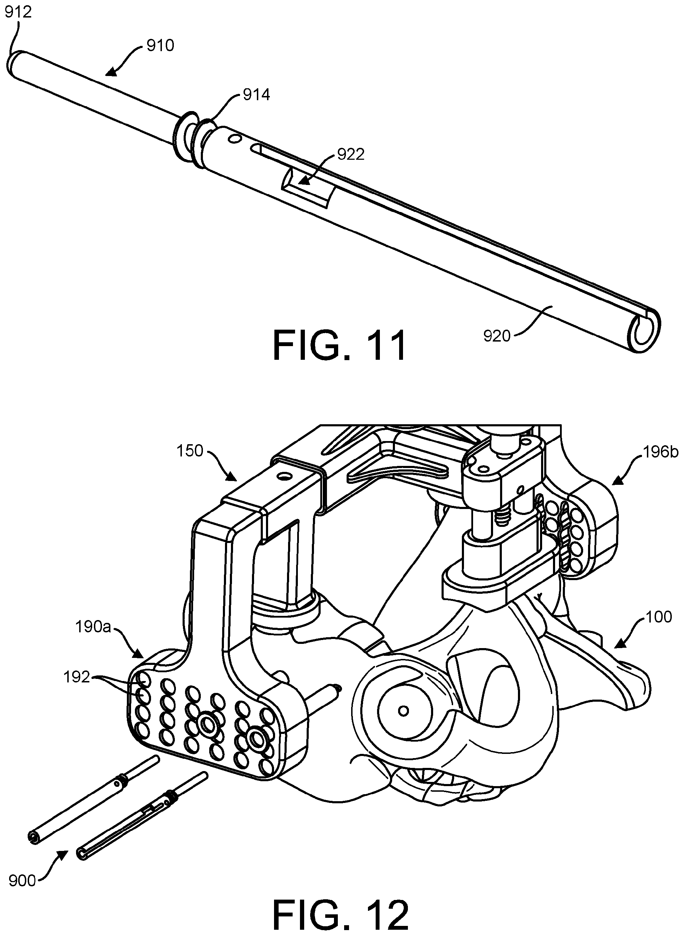

With reference to FIG. 12, a mechanism of securing the pin-screw assembly 900 to the pelvis 100 can be used. The surgeon advances the pin-screw assembly 900 through one of the guides 192 provided by the pelvic navigation frame 150 (either through a percutaneously placed cannula within the soft tissue or directly through the soft-tissue envelope) directly to the outer surface of the hemipelvis. Striking the back of the pin driver handle with a mallet (with or without rotation of the handle), the pin screw assembly 900 (in state in which the externally threaded screw component is only partially threaded onto the distal pin segment) is advanced through the outer pelvic table and either into or through the inner table.

Once the pin is advanced to the depth of the inner table, the pin handle is held securely (resisting transmitted rotational loading of the pin) while the threaded segment is advanced over the pin with the rotation of its associated cannulated insertion instrument handle. Once the threaded segment of the assembly is fully thread engaged onto the distal pin segment of the assembly, the handles are removed and the pin-screw assembly is subsequently utilized for positioning posts within the features of the cannulated screw segment for various measurement purposes (e.g. multiaxial reference sensor, leg length and trochanteric off-set measuring instrument).

Interference fit of various means between the drivers and assembly components are anticipated, including friction fit, slotting of either the driver or driven component for collapsing and/or splaying of either to increase the dissociation force required to separate driver from driven component.

While this specification contains many specifics, these should not be construed as limitations on the scope of an invention that is claimed or of what may be claimed, but rather as descriptions of features specific to particular embodiments. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or a variation of a sub-combination. Similarly, while operations are depicted in the drawings in a particular order, this should not be understood as requiring that such operations be performed in the particular order shown or in sequential order, or that all illustrated operations be performed, to achieve desirable results. Only a few examples and implementations are disclosed. Variations, modifications and enhancements to the described examples and implementations and other implementations may be made based on what is disclosed. The scope of the invention is defined by the appended claims.

## Claims

1. A device comprising:
an adjustable frame (150) comprising three support stanchions (160, 170, 180), the first and second support stanchions (160, 170) of the three support stanchions (160, 170, 180) being configured to rest on cutaneous surfaces immediately superficial to anterior superior iliac spine prominences (120, 130), and the third stanchion (180) of the three support stanchions (160, 170, 180) being configured to be supported by the skin and subcutaneous tissue immediately superficial to the symphysis pubis (140);
the device being **characterized by**:
a surgical guide (190a, 190b) associated with the adjustable frame; and
at least two guide pins (110a, 110b),
wherein the surgical guide (190a, 190b) is configured to position the at least two guide pins (110a, 110b) in the lateral pelvis superior to the acetabular cavity and comprises one or more guide holes (192) for such positioning, and
wherein the at least two guide pins (110a, 110b) are configured to be inserted into the pelvis (100) along axes that are each parallel with a line defined by the anterior superior iliac spine prominences (120, 130), such that a plane containing both of the pin axes is parallel or angularly referenced to an anterior pelvic plane (125) defined by the anterior superior iliac spine prominences (120, 130) and the symphysis pubis (140).

2. The device of claim 1, wherein the surgical guide (190a, 190b) is coupled to at least one of the first and second support stanchions (160, 170) and the at least two guide holes (192) are sized to receive and position the at least two guide pins (110a, 110b) into a lateral wall of an ilium of the patient superior to an acetabular socket.

3. The device of claim 2, wherein a configuration of the three support stanchions (160, 170, 180) provides a known angular relationship to the one or more guide pins (110a, 110b) within at least one anatomic plane.

4. The device of claim 3, wherein the known angular relationship comprises that of an acetabular inclination and/or an anteversion.

5. The device of claim 1, further comprising a mechanical guide (310) configured to secure a screw (745) in a floor of the acetabulum of the patient.

6. The device of claim 5, wherein the mechanical guide (310) has an attached radially positioned ring configuration configured to aid in a drill placement within the acetabular socket of the patient.

7. The device of claim 1, wherein each support stanchion of the support stanchions (160, 170, 180) comprises a concave interior surface configured to stabilize the adjustable frame (150).

8. The device of claim 1, wherein one or more of the support stanchions (160, 170, 180) comprise a channel (520) extending through the support stanchion (160, 170, 180), the channel (520) sized to receive a depth gauge pin probe (510).

9. The device of claim 1, wherein a first axis between the first and second stanchions (160, 170), a second axis between the first stanchion (160) and the third stanchion (180), and a third axis between the second stanchion (170) and the third stanchion (180) collectively define the anterior pelvic plane (125).

10. The device of claim 9, wherein the third support stanchion (180) comprises an adjustment component (530) configured to adjust a height of the third support stanchion (180) and the anterior pelvic plane (125).

11. The device of claim 1 and a table rail attachment, wherein the adjustable frame (150) is configured to be compressed against the patient using the table rail attachment, via which compression of the pelvis (100) of the patient against the table is accomplished.

12. The device of claim 1, further comprising an axial guide (610) configured to be coupled to the at least two guide pins (110a, 110b), the axial guide (610) having a guide axis along an axis of an operative limb and that extends over a greater trochanter of the operative limb, the axial guide (610) being configured to determine limb length and hip offset by measuring a distance to a third pin (620) fixed to the greater trochanter of the operative limb.

13. The device of claim 12, wherein the adjustable axial guide (610) further comprises a guide channel (640) having an axis that is orthogonal to the guide axis and generally parallel to the at least two guide pins (110a, 110b).

14. The device of claim 13, wherein the adjustable axial guide (610) further comprises a plurality of graduations (612) along the guide axis, the graduations (612) providing distance information between the at least two guide pins (110a, 110b) and the third pin (620).

15. The device of claim 13, wherein the guide channel (640) is configured to receive a screwdriver (630) associated with the third pin (620) for implantation in the greater trochanter of the operative limb.

## Patentansprüche

1. Vorrichtung, umfassend:
einen verstellbaren Rahmen (150), der drei Stützstreben (160, 170, 180) umfasst, wobei die erste und die zweite Stützstrebe (160, 170) der drei Stützstreben (160, 170, 180) konfiguriert sind, auf Hautoberflächen aufzuliegen, die direkt oberflächlich zu vorderen oberen Darmbeinstachelvorsprüngen (120, 130) sind, und die dritte Strebe (180) der drei Stützstreben (160, 170, 180) so konfiguriert ist, dass sie von der Haut und dem Subkutangewebe unmittelbar oberflächlich zu der Schambeinfuge (140) getragen wird;
wobei die Vorrichtung **gekennzeichnet ist durch**:
eine chirurgische Führung (190a, 190b), die dem verstellbaren Rahmen zugeordnet ist; und
mindestens zwei Führungsstifte (110a, 110b),
wobei die chirurgische Führung (190a, 190b) konfiguriert ist, die mindestens zwei Führungsstifte (110a, 110b) in dem lateralen Becken oberhalb der Hüftgelenkpfannenkavität zu positionieren, und ein oder mehrere Führungslöcher (192) für diese Positionierung umfasst, und
wobei die mindestens zwei Führungsstifte (110a, 110b) konfiguriert sind, entlang von Achsen in das Becken (100) eingeführt zu werden, die jeweils parallel zu einer Linie sind, die **durch** die vorderen oberen Darmbeinstachelvorsprünge (120, 130) definiert ist, sodass eine Ebene, die beide Stiftachsen enthält, parallel oder schräg in Bezug auf eine vordere Beckenebene (125) ist, die **durch** die vorderen oberen Darmbeinstachelvorsprünge (120, 130) und die Schambeinfuge (140) definiert ist.

2. Vorrichtung nach Anspruch 1, wobei die chirurgische Führung (190a, 190b) mit mindestens einer von der ersten und der zweiten Stützstrebe (160, 170) gekoppelt ist und die mindestens zwei Führungslöcher (192) so bemessen sind, dass sie die mindestens zwei Führungsstifte (110a, 110b) aufnehmen und in einer Seitenwand eines Darmbeins des Patienten oberhalb einer Hüftgelenkpfannenhöhle positionieren.

3. Vorrichtung nach Anspruch 2, wobei eine Konfiguration der drei Stützstreben (160, 170, 180) eine bekannte Winkelbeziehung zu dem einen oder den mehreren Führungsstiften (110a, 110b) innerhalb mindestens einer anatomischen Ebene bereitstellt.

4. Vorrichtung nach Anspruch 3, wobei die bekannte Winkelbeziehung die einer Hüftgelenkpfannenneigung und/oder einer Anteversion umfasst.

5. Vorrichtung nach Anspruch 1, die ferner eine mechanische Führung (310) umfasst, die konfiguriert ist, eine Schraube (745) in einem Boden der Hüftgelenkpfanne des Patienten zu befestigen.

6. Vorrichtung nach Anspruch 5, wobei die mechanische Führung (310) eine angebrachte, radial positionierte Ringkonfiguration aufweist, die so konfiguriert ist, dass sie eine Bohrerplatzierung innerhalb der Hüftgelenkpfannenhöhle des Patienten unterstützt.

7. Vorrichtung nach Anspruch 1, wobei jede Stützstrebe der Stützstreben (160, 170, 180) eine konkave Innenfläche aufweist, die so konfiguriert ist, dass sie den verstellbaren Rahmen (150) stabilisiert.

8. Vorrichtung nach Anspruch 1, wobei eine oder mehrere der Stützstreben (160, 170, 180) einen Kanal (520) umfassen, der sich durch die Stützstrebe (160, 170, 180) erstreckt, wobei der Kanal (520) zur Aufnahme einer Tiefenmessstiftsonde (510) bemessen ist.

9. Vorrichtung nach Anspruch 1, wobei eine erste Achse zwischen der ersten und der zweiten Strebe (160, 170), eine zweite Achse zwischen der ersten Strebe (160) und der dritten Strebe (180) und eine dritte Achse zwischen der zweiten Strebe (170) und der dritten Strebe (180) gemeinsam die vordere Beckenebene (125) definieren.

10. Vorrichtung nach Anspruch 9, wobei die dritte Stützstrebe (180) eine Einstellkomponente (530) umfasst, die konfiguriert ist, eine Höhe der dritten Stützstrebe (180) und der vorderen Beckenebene (125) einzustellen.

11. Vorrichtung nach Anspruch 1 und eine Tischschienenbefestigung, wobei der verstellbare Rahmen (150) konfiguriert ist, mit Hilfe der Tischschienenbefestigung gegen den Patienten gedrückt zu werden, wodurch eine Kompression des Beckens (100) des Patienten entgegen dem Tisch erreicht wird.

12. Vorrichtung nach Anspruch 1, die ferner eine axiale Führung (610) umfasst, die konfiguriert ist, mit den mindestens zwei Führungsstiften (110a, 110b) gekoppelt zu werden, wobei die axiale Führung (610) eine Führungsachse entlang einer Achse einer zu operierenden Gliedmaße aufweist und sich über einen Trochanter major der zu operierenden Gliedmaße erstreckt, wobei die axiale Führung (610) konfiguriert ist, eine Gliedmaßenlänge und einen Hüftversatz durch Messen eines Abstands zu einem dritten Stift (620) zu bestimmen, der an dem Trochanter major der zu operierenden Gliedmaße befestigt ist.

13. Vorrichtung nach Anspruch 12, wobei die einstellbare axiale Führung (610) ferner einen Führungskanal (640) umfasst, der eine Achse aufweist, die orthogonal zu der Führungsachse und im Allgemeinen parallel zu den mindestens zwei Führungsstiften (110a, 110b) ist.

14. Vorrichtung nach Anspruch 13, wobei die einstellbare axiale Führung (610) ferner eine Vielzahl von Einteilungen (612) entlang der Führungsachse aufweist, wobei die Einteilungen (612) Abstandsinformationen zwischen den mindestens zwei Führungsstiften (110a, 110b) und dem dritten Stift (620) bereitstellen.

15. Vorrichtung nach Anspruch 13, wobei der Führungskanal (640) konfiguriert ist, einen Schraubendreher (630), der dem dritten Stift (620) zugeordnet ist, zur Implantation in den Trochanter major der zu operierenden Gliedmaße aufzunehmen.

## Revendications

1. Dispositif comprenant :
un cadre réglable (150) comprenant trois montants de support (160, 170, 180), les premier et deuxième montants de support (160, 170) des trois montants de support (160, 170, 180) étant configurés pour reposer sur des surfaces cutanées immédiatement superficielles aux protubérances de l'épine iliaque antéro-supérieure (120, 130), et le troisième montant (180) des trois montants de support (160, 170, 180) étant configuré pour être supporté par la peau et le tissu sous-cutané immédiatement superficiel à la symphyse pubienne (140) ;
le dispositif étant **caractérisé par** :
un guide chirurgical (190a, 190b) associé au cadre réglable ; et
au moins deux broches de guidage (110a, 110b),
dans lequel le guide chirurgical (190a, 190b) est configuré pour positionner les au moins deux broches de guidage (110a, 110b) dans le bassin latéral au-dessus de la cavité acétabulaire et comprend un ou plusieurs trous de guidage (192) pour un tel positionnement, et
dans lequel les au moins deux broches de guidage (110a, 110b) sont configurées pour être insérées dans le bassin (100) le long d'axes qui sont chacun parallèles à une droite définie par les protubérances de l'épine iliaque antéro-supérieure (120, 130), de sorte qu'un plan contenant les deux axes de broche est parallèle ou est référencé angulairement par rapport à un plan pelvien antérieur (125) défini par les protubérances de l'épine iliaque antéro-supérieure (120, 130) et la symphyse pubienne (140).

2. Dispositif selon la revendication 1, dans lequel le guide chirurgical (190a, 190b) est couplé à au moins l'un des premier et deuxième montants de support (160, 170) et les au moins deux trous de guidage (192) sont dimensionnés pour recevoir et positionner les au moins deux broches de guidage (110a, 110b) dans une paroi latérale d'un ilium du patient au-dessus d'une cavité acétabulaire.

3. Dispositif selon la revendication 2, dans lequel une configuration des trois montants de support (160, 170, 180) fournit une relation angulaire connue par rapport aux une ou plusieurs broches de guidage (110a, 110b) dans au moins un plan anatomique.

4. Dispositif selon la revendication 3, dans lequel la relation angulaire connue comprend celle d'une inclinaison acétabulaire et/ou d'une antéversion.

5. Dispositif selon la revendication 1, comprenant en outre un guide mécanique (310) configuré pour fixer une vis (745) dans un plancher du cotyle du patient.

6. Dispositif selon la revendication 5, dans lequel le guide mécanique (310) a une configuration annulaire fixée positionnée radialement configurée pour aider à un placement de foret à l'intérieur de la cavité acétabulaire du patient.

7. Dispositif selon la revendication 1, dans lequel chaque montant de support des montants de support (160, 170, 180) comprend une surface intérieure concave configurée pour stabiliser le cadre réglable (150).

8. Dispositif selon la revendication 1, dans lequel un ou plusieurs des montants de support (160, 170, 180) comprennent un canal (520) s'étendant à travers le montant de support (160, 170, 180), le canal (520) étant dimensionné pour recevoir une sonde à tige de mesure de profondeur (510).

9. Dispositif selon la revendication 1, dans lequel un premier axe entre les premier et deuxième montants (160, 170), un deuxième axe entre le premier montant (160) et le troisième montant (180), et un troisième axe entre le deuxième montant (170) et le troisième montant (180) définissent collectivement le plan pelvien antérieur (125) .

10. Dispositif selon la revendication 9, dans lequel le troisième montant de support (180) comprend un composant de réglage (530) configuré pour régler une hauteur du troisième montant de support (180) et du plan pelvien antérieur (125).

11. Dispositif selon la revendication 1 et fixation de rail de table, dans lesquels le cadre réglable (150) est configuré pour être comprimé contre le patient à l'aide de la fixation de rail de table, par l'intermédiaire de laquelle la compression du bassin (100) du patient contre la table est accomplie.

12. Dispositif selon la revendication 1, comprenant en outre un guide axial (610) configuré pour être couplé aux au moins deux broches de guidage (110a, 110b), le guide axial (610) ayant un axe de guidage le long d'un axe d'un membre opérationnel et qui s'étend sur un grand trochanter du membre fonctionnel, le guide axial (610) étant configuré pour déterminer la longueur du membre et le décalage de la hanche en mesurant une distance jusqu'à une troisième broche (620) fixée au grand trochanter du membre fonctionnel.

13. Dispositif selon la revendication 12, dans lequel le guide axial réglable (610) comprend en outre un canal de guidage (640) ayant un axe qui est orthogonal à l'axe de guidage et généralement parallèle aux au moins deux broches de guidage (110a, 110b).

14. Dispositif selon la revendication 13, dans lequel le guide axial réglable (610) comprend en outre une pluralité de graduations (612) le long de l'axe de guidage, les graduations (612) fournissant des informations de distance entre les au moins deux broches de guidage (110a, 110b) et la troisième broche (620).

15. Dispositif selon la revendication 13, dans lequel le canal de guidage (640) est configuré pour recevoir un tournevis (630) associé à la troisième broche (620) pour une implantation dans le grand trochanter du membre fonctionnel.
